# EUROPEAN PATENT APPLICATION

(11) **EP 4 212 000 A1**
(43) Date of publication of application: **19.07.2023**
(21) Application number: 21866089.2
(22) Date of filing: 13.09.2021
(51) Int. Cl.: A01B 79/02, A01N 59/00, A01P 21/00

(54) **METHOD FOR INCREASING CONTENT AND GENE EXPRESSION OF AROMATIC SUBSTANCE IN FRUITS AND VEGETABLES BY USING HYDROGEN RICH WATER**

(30) Priority: 14.09.2020 CN 202010959454; 13.07.2021 CN 202110791343; 13.07.2021 CN 202110791514
(71) Applicant: Nanjing Agricultural University, Nanjing, Jiangsu 210095 (CN); L'AIR LIQUIDE, SOCIETE ANONYME POUR L'ETUDE ET L'EXPLOITATION DES PROCEDES GEORGES CLAUDE, 75007 Paris (FR)
(72) Inventor: SHEN, Wenbiao, Nanjing, Jiangsu 210095 (CN); ZENG, Yan, Shanghai 201108 (CN); LI, Longna, Nanjing, Jiangsu 210095 (CN); SUNDAR, M, Newark, Delaware 19702 (US); WANG, Shu, Nanjing, Jiangsu 210095 (CN); CHENG, Xu, Shanghai 201108 (CN); LIU, Yuhao, Nanjing, Jiangsu 210095 (CN)
(74) Representative: Air Liquide
(86) International application number: PCT/CN2021/117890
(87) International publication number: WO 2022/053037

(57) **Abstract**

A method for increasing the content and gene expression of an aromatic substance in fruits and vegetables by using hydrogen rich water. Hydrogen rich water can be used in combination with a certain amount of chemical fertilizers and pesticides, hydrogen rich water is used to irrigate farmland, and during the period of irrigation, a hydrogen concentration measured at an outlet of hydrogen rich water is kept not lower than a set value.

## Description

### Technical Field

The present invention relates to the technical field of agriculture, in particular to a method for irrigating fields with hydrogen-rich water. The method can also use hydrogen-rich water, pesticides and chemical fertilizers in combination at the same time, to maintain or increase the content of aromatic substances in fruits and vegetables and the gene expression of aromatic substances.

### Background Art

Fruits and vegetables are important parts of the diet of humans. In recent years, as consumers' demands for flavours have diversified, the amount of attention paid to volatile aromatic substances in fruits and vegetables has gradually increased. Volatile flavour substances in fruits and vegetables mainly include aldehydes, ketones, esters, alcohols, terpenoids, aromatic-group compounds, aliphatic compounds and heterocyclic compounds, etc. Conventional planting methods, despite increasing the yield of agricultural products, hinder the conversion and synthesis of volatile substances within the body of fruits and vegetables, due to excessive use of chemical fertilizers and pesticides, thus weakening the quality and natural flavour of the agricultural products.

Hydrogen has various biological effects as well as being safe and economical to use, so has very broad prospects for application in agricultural production. It is known that hydrogen has various boosting effects on plant growth and development. As an important signalling molecule, hydrogen can promote the sprouting of rye, mung beans, paddy and other seeds; promote the development of nepenthes, marigold and cucumber explant adventitious roots; and increase plants' resistance to disease, insects, drought and salt, etc. At the same time, hydrogen can increase the content of certain secondary metabolites, e.g. increase the content of anthocyanins and polyphenols in radish sprouts and strawberries, thereby improving quality. However, at present, no reports of the effects of hydrogen on volatile flavour substances in fruits and vegetables have yet been found.

In the prior art, reports on the effects of hydrogen on crops are mostly seen in water cultivation and soil cultivation in pots at the laboratory scale. For example, in the Chinese invention patent "A hydrogen-rich liquid plant growth regulator and preparation method and use thereof' (patent no. ZL201210154005.0), it is recorded that hydrogen is passed directly into a nutritional liquid to obtain a hydrogen-rich plant growth regulator. That invention disclosed the preparation of hydrogen by a fermentation method, an electrolysis method, a chemical method and a cylinder, followed by the dissolution of hydrogen in water or a nutrient liquid, thereby producing the plant growth regulator rich in hydrogen. Such a hydrogen-rich plant growth regulator can increase yield and improve quality, as well as increasing the stress tolerance/resistance of plants.

In the laboratory, the use of hydrogen-rich water sticks or the passage of pure hydrogen into water, etc. to produce hydrogen-rich water is sufficient for scenarios requiring small amounts of hydrogen-rich water. In water cultivation and soil cultivation in pots at the laboratory scale, etc., the cultivation conditions are easy to control, and the observed phenomena are obvious, but in such cultivation, the object of research is often a single factor or a few controllable factors, which are easy to manage. Field agriculture mainly refers to crops that are planted on large swathes of farmland, the chief difference of field agriculture being the fact that the soil in fields is closer to the complex environment of actual fields, with poor controllability, imprecision and difficulty of management; research results obtained in the laboratory are often unable to be copied and reproduced in fields, and for this reason, research into the nature and conditions of planting of crops in fields is unique, with a higher level of difficulty.

### Summary of the Invention

An objective of the present invention is to provide a method for irrigating fields with hydrogen-rich water, to increase the content of aromatic substances in fruits and vegetables and the gene expression thereof, while overcoming shortcomings of hydrogen-rich water prepared by existing hydrogen production techniques, such as a short half-life and low concentration. Irrigation of fields with hydrogen-rich water can bring into play the biological effects of highly concentrated hydrogen while meeting the normal demand of crops for water, to achieve the objectives of increasing the content of aromatic substances in fruits and vegetables and enhancing the fragrance of fruits and vegetables.

A first aspect of the present invention discloses a method for using hydrogen-rich water to increase the content of aromatic substances in fruits and vegetables, wherein an outlet hydrogen concentration of hydrogen-rich water is kept no lower than a set value within a period of time for which a field is irrigated with hydrogen-rich water.

Further, the period of time for which the field is irrigated is at least 2 hours.

Further, the amount of the hydrogen-rich water used to irrigate the field accounts for 30% or more of the total amount of water used to irrigate the field.

Further, the hydrogen-rich water is nanobubble hydrogen water.

Further, the aromatic substances comprise ester substances, ketone substances and alcohol substances.

Further, the set value of the outlet hydrogen concentration of the hydrogen-rich water is 500 ppb, preferably 600 ppb, more preferably 700 ppb, and most preferably 1000 ppb.

Further, the amount of the hydrogen-rich water used to irrigate the field is 0.1 - 500 cubic metres per mu.

Further, the alcohol substances comprise one or both of nerolidol and linalool.

Further, the ketone substances comprise DMMF.

Further, the ester substances comprise ethyl hexanoate.

Further, the fruits and vegetables comprise one or both of fruit and vegetable crops with aromatic fragrances, and preferably comprise berries.

A second aspect of the present invention discloses a method for using hydrogen-rich water to increase gene expression of aromatic substances of fruits and vegetables, wherein an outlet hydrogen concentration of hydrogen-rich water is kept no lower than a set value within a period of time for which a field is irrigated with hydrogen-rich water.

Further, the genes comprise strawberry lipoxygenase gene *FaLOX,* strawberry O-methyltransferase gene *FaOMT* and nerolidol synthase gene *FaNES1.*

Another objective of the present invention is to provide a method for using hydrogen-rich water, pesticide and chemical fertilizer in combination to maintain or increase the content of aromatic substances in fruits and vegetables and the gene expression thereof, while overcoming shortcomings of hydrogen-rich water prepared by existing hydrogen production techniques, such as a short half-life and low concentration. Irrigation of fields with hydrogen-rich water can bring into play the biological effects of highly concentrated hydrogen while meeting the normal demand of crops for water, increasing crop yield and reducing disease and pests, to achieve the objectives of maintaining or increasing the content of aromatic substances in fruits and vegetables and enhancing the fragrance of fruits and vegetables.

A third aspect of the present invention provides a method for using hydrogen-rich water, pesticide and chemical fertilizer in combination to maintain or increase the content of aromatic substances in fruits and vegetables: an outlet hydrogen concentration of hydrogen-rich water is kept no lower than a set value within a period of time for which a field is irrigated with hydrogen-rich water,
wherein chemical fertilizer and pesticide are applied to the field in the following doses:
one of, or a combination of more than one of, the following pesticides is sprayed within a growth period of the fruits and vegetables:
pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40-50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu;
the chemical fertilizer application plan is as follows: organic fertilizer 1000 - 1100 kg/mu, compound fertilizer 70 - 80 kg/mu, bacterial manure 4 - 5 kg/mu.

Further, the set value is 500 ppb, preferably 700 ppb, more preferably 1000 ppb, most preferably 1500 ppb.

Further, the period of time for which the field is irrigated is at least 2 hours.

Further, the amount of the hydrogen-rich water used to irrigate the field accounts for 30% or more of the total amount of water used to irrigate the field.

Further, the hydrogen-rich water is nanobubble hydrogen water.

Further, the amount of the hydrogen-rich water used to irrigate the field is 0.1 - 500 cubic metres per mu.

Further, the aromatic substances comprise ester substances, ketone substances and alcohol substances.

Further, the alcohol substances comprise one or both of nerolidol and linalool.

Further, the ketone substances comprise DMMF.

Further, the ester substances comprise ethyl hexanoate.

Further, the fruits and vegetables comprise fruit and vegetable crops with aromatic fragrances, and preferably comprise berries.

A fourth aspect of the present invention provides a method for using hydrogen-rich water, pesticide and chemical fertilizer in combination to maintain or increase the content of aromatic substances in fruits and vegetables: within a period of time for which a field is irrigated with hydrogen-rich water, the ratio of an outlet hydrogen concentration of hydrogen-rich water to an amount of chemical fertilizer applied is kept greater than 0.63 ppb/kg, preferably greater than 0.89 ppb/kg, more preferably greater than 1.27 ppb/kg, and most preferably greater than 1.90 ppb/kg;
one of, or a combination of more than one of, the following pesticides is sprayed within a growth period of the fruits and vegetables: pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40 - 50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu.

A fifth aspect of the present invention provides a method for using hydrogen-rich water, pesticide and chemical fertilizer in combination to maintain or increase gene expression of aromatic substances in fruits and vegetables: an outlet hydrogen concentration of hydrogen-rich water is kept no lower than a set value within a period of time for which a field is irrigated with hydrogen-rich water,
wherein chemical fertilizer and pesticide are applied to the field in the following doses:
one of, or a combination of more than one of, the following pesticides is sprayed within a growth period of the fruits and vegetables:
pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40 - 50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu;
the chemical fertilizer application plan is as follows: organic fertilizer 1000 - 1100 kg/mu, compound fertilizer 70 - 80 kg/mu, bacterial manure 4 - 5 kg/mu.

Further, the genes comprise strawberry O-methyltransferase gene *FaOMT* and nerolidol synthase gene *FaNES1.*

Compared with the prior art, the technical solution provided in the present invention has the following advantages:
1. Irrigation with hydrogen water at a specific concentration can increase the content of various aromatic substances in fruits and vegetables, and increase the accumulation of volatile substances in fruits and vegetables, while enhancing the relative expression level of relevant regulatory genes, and enhancing the completeness of flavour of fruits and vegetables. When used in combination with pesticide and chemical fertilizer, adverse effects of the pesticide and chemical fertilizer on the flavour of the fruits and vegetables can be mitigated.
2. The hydrogen in nanobubble hydrogen water is dissolved to the greatest extent possible, with a longer residence time in water and a longer half-life, so is more suitable for actual situations in field production in which the irrigation area is large and irrigation takes a long time.
3. Hydrogen water may be used as normal irrigation water, is non-irritating to the human body, has a high level of safety, diffuses quickly after irrigation of fields, and is far lower than the minimum limit for hydrogen explosion (4%).
4. Hydrogen water is composed of only hydrogen and water, has stable chemical properties, is non-polluting and environmentally friendly, so will not have an adverse impact on the human body or the environment.

### Brief Description of the Drawings

Fig. 1 shows a schematic chart of the trend of variation of a specific ester substance in strawberries with hydrogen-rich water concentration when no chemical fertilizer or pesticide is applied.
Fig. 2 shows a schematic chart of the trend of variation of a specific alcohol substance in strawberries with hydrogen-rich water concentration when no chemical fertilizer or pesticide is applied.
Fig. 3 shows a schematic chart of the trend of variation of nerolidol and nerolidol synthase gene FaNES 1 expression in strawberries with hydrogen-rich water concentration when no chemical fertilizer or pesticide is applied.
Fig. 4 shows a schematic chart of the trend of variation of DMMF and O-methyltransferase gene (*FaOMT*) expression in strawberries with hydrogen-rich water concentration when no chemical fertilizer or pesticide is applied.
Fig. 5 shows a schematic chart of the trend of variation of the total content of aromatic substances in strawberries with hydrogen-rich water concentration when chemical fertilizer and pesticide are applied.
Fig. 6 shows a schematic chart of the trend of variation of trans-2-hexenal, *FaLOX* and the total content of aldehyde substances in strawberries with hydrogen-rich water concentration when chemical fertilizer and pesticide are applied.
Fig. 7 shows a schematic chart of the trend of variation of alcohol substances, nerolidol, linalool and *FaNES1* in strawberries with hydrogen-rich water concentration when chemical fertilizer and pesticide are applied.

### Preferred Embodiments of the Invention

Specific embodiments of the present invention are explained in detail below in conjunction with the accompanying drawings. However, the present invention should be understood to not be limited to embodiments such as those described below, and the technical concept of the present invention may be implemented in combination with other well-known technologies or other technologies having the same function as those well-known technologies.

In addition, the terms "first" and "second" are merely used for descriptive purposes, and must not be interpreted as indicating or implying relative importance or implicitly specifying the quantity of the technical feature indicated. Thus, features for which "first" and "second" are defined may explicitly or implicitly include one or more of said feature. In the description of the present invention, the meaning of "multiple" is two or more, unless clearly and specifically specified otherwise.

Unless clearly indicated otherwise, each aspect or embodiment defined herein may be combined with any other aspect(s) or embodiment(s). In particular, any preferred or advantageous feature(s) indicated can be combined with any other preferred or advantageous feature(s) indicated.

### Explanation of terms

As used herein, the "nanobubbles" in "nanobubble hydrogen water" may be understood as being bubbles of diameter 10 - 500 nm; the nanobubbles may have an average diameter of less than 500 nm, or an average diameter of about 10 nm to about 500 nm, or about 75 nm to about 200 nm. The hydrogen concentration of the nanobubble hydrogen water may reach 500 - 1500 ppb. In some embodiments, these nanobubbles can be stable for at least about 15 hours in a liquid carrier at ambient pressure and temperature.

As used herein, the solubility of hydrogen means the number of volumes of hydrogen (at a pressure of 1 standard atmosphere) dissolved in 1 volume of water at a given temperature. Under standard conditions, i.e. one atmosphere and 20°C, the solubility of hydrogen is 1.83% (1.83 ml of hydrogen can be dissolved in every 100 ml of water, and the volume ratio and mass ratio can be obtained by conversion, i.e. 1.6 ppm).

As used herein, "hydrogen-rich water" (HRW) means water that is rich in hydrogen. In an environment of one atmosphere and 20°C, the maximum concentration of hydrogen dissolved in water is 1.6 ppm (i.e. 1600 ppb), i.e. at most 1.6 mg of hydrogen dissolves in each kg of water, at which time the saturation concentration is reached.

As used herein, "half-life" means the time required for the concentration to fall by half. After dissolving in water, hydrogen will still leave the water slowly in an open vessel, causing the hydrogen concentration of the water to gradually decrease - this is referred to as "escaping from solution". In an open vessel, the hydrogen half-life of ordinary hydrogen water is about 1 - 2 hours, while the hydrogen half-life of nanobubble hydrogen water is about 3 - 8 hours depending on the concentration.

As used herein, the "outlet hydrogen concentration of hydrogen-rich water" means the dissolved hydrogen concentration measured at a hydrogen-rich water outlet. Even taking into account hydrogen escape, those skilled in the art know that methods such as the continuous addition of hydrogen water may be used to keep the concentration of hydrogen water irrigating the fields as close as possible to the outlet concentration, e.g. an outlet concentration of 80% or more, preferably an outlet concentration of 85% or more, more preferably an outlet concentration of 90% or more, most preferably an outlet concentration of 95% - 99.9%.

As used herein, the "period of time for which the field is irrigated" means that within a period of time, the concentration of hydrogen water irrigating the field is kept above the minimum value required by the present invention through the use of hydrogen-rich water. This period of time may be continuous or interrupted.

To increase the solubility and residence time of hydrogen in water, people combine nanobubble technology and hydrogen-rich water; using hydrogen produced by electrolysis or hydrogen from cylinders as a gas source, a nanobubble generating module is used to isolate nano-scale pure hydrogen bubbles, which are then dissolved in water to obtain nanobubble hydrogen water. By comparison, the dissolved hydrogen in hydrogen-rich water prepared by introducing hydrogen directly into water has a very short half-life, only about 1 - 2 hours, so the residence time of hydrogen is very short; after being prepared, such water must be used immediately, otherwise hydrogen will escape. Nanobubble hydrogen water has a longer half-life, so is suitable for actual situations in field production in which the irrigation area is large and irrigation takes a long time.

As used herein, the term "pesticide" means at least one active substance selected from fungicides, insecticides, nematicides, herbicides, safeners, biological pesticides and/or growth regulators. In one embodiment, the pesticide is an insecticide. The term "pesticide components" is meant to include chemicals for crop protection or a mixture of these chemicals. More specifically, each component is selected from herbicides, fungicides, microbicides, insecticides, insect repellents, acaricides, miticides, nematicides and plant growth regulators, etc. The term "insecticide" is intended to indicate a substance used to attract, lure, destroy or alleviate any insect pest. Insecticides are a type of biocide. The most common use of pesticides is as plant protection products (also called crop protection products), which generally protect plants from the harmful effects of weeds, plant diseases or insects for example, including but not limited to herbicides, insecticides, insect growth regulators, nematicides, termiticides, molluscicides, fish-killing agents, bird-killing agents, rodenticides, poisons, bactericides, insect repellents, animal repellents, antimicrobial agents, fungicides, disinfectants (antimicrobial agents) and disinfecting microbicides.

As used herein, the term "chemical fertilizer" or "chemical fertilizer substance" is meant to indicate any product used in agriculture and/or horticulture, aimed at creating, reorganizing, protecting or increasing the productive capacity of an area of ground, providing one or more nutritional components for use by plants, regardless of whether the form of composition of the product is particulate, powdered or liquid. Of course, the term "chemical fertilizer substance" also includes fertilizers, soil improvers and/or soil improving substances.

As used herein, the term "field" means ground used in agricultural production or a tilled field, including but not limited to land or farmland planted with cereal crops, cash crops (oil crops, vegetable crops, flowers, pasture, fruit trees), industrial raw material crops, feed crops or Chinese traditional medicine materials; preferably, it means land planted with crops that can fully grow in large quantities or be harvested over a large area and are used for profit or provisions (e.g. grains, vegetables, cotton, flax, etc.); more preferably, it means farmland or land planted with paddy, maize, beans, root crops, highland barley, broad beans, wheat, oilseed, turnip, mustard, peanuts, sesame, hemp, sunflower, radish, Chinese cabbage, celery, Chinese chives, garlic, onion, carrots, snake melon, cabbage, Jerusalem artichoke, sword beans, coriander, celtuce, citron daylily, chilli, cucumber, tomatoes, coriander, etc. In the present invention, the term "farmland" is equivalent to "field", and there are no particular requirements for the area or size/shape of the farmland or fields.

For a method for determining the fragrance components of agricultural products, see the reference document: Zhang Y, Wang G, Dong J, *et al.* (ZHANG Yuntao, WANG Guixia, DONG Jing, et al.) Analysis of volatile compounds in fruits of 33 European and American strawberry varieties. Journal of Fruit Science, 2011, 28(3): 438-442.

Hydrogen-rich water prepared by electrolysis: a hydrogen generator (SHC-300, Saikesaisi, Shandong, China) uses 2 - 24 V DC electricity to electrolyse water, hydrogen is obtained after vapour separation and drying, and then passed into aqueous solution for 60 minutes to obtain hydrogen-rich water prepared by electrolysis.

Hydrogen-rich water prepared by cylinder: hydrogen from a hydrogen cylinder is passed into water to obtain hydrogen-rich water prepared from cylinder hydrogen.

Nano hydrogen-rich water prepared by electrolysis: a hydrogen generator (CA/H, Cawolo, Guangdong, China) uses 7 - 21 V DC electricity to electrolyse water, hydrogen is obtained after vapour separation and drying, then passed into water through a nano aeration head, to obtain nano hydrogen-rich water prepared by electrolysis.

Nano hydrogen-rich water prepared by cylinder: hydrogen from a hydrogen cylinder is passed into water through a nano aeration head to obtain nano hydrogen-rich water prepared by cylinder.

A Dissolved Hydrogen Meter ENH-2000 (TRUSTLEX, Japan) that has been calibrated by gas chromatography is used to determine the concentration of dissolved hydrogen in hydrogen-rich water or nanobubble hydrogen water prepared by electrolysis or prepared from cylinder gas.

### Embodiment 1:

This embodiment takes field production of strawberries as an example; the "Hongyan" strawberry seeds chosen were purchased at Shanghai City Seed Market. The "Hongyan" strawberry seeds were sown in fields, the area of each field being 467 square metres (about 0.7 mu). Each field was treated as follows:
Field no. 1: irrigation with ordinary water, no chemical fertilizer or pesticide applied.
Field no. 3: irrigation with hydrogen-rich water prepared by electrolysis (during irrigation, the outlet hydrogen concentration of the hydrogen-rich water is about 300 ppb, and the half-life thereof is about 1 hour), no chemical fertilizer or pesticide applied.
Field no. 5: irrigation with nanobubble hydrogen water prepared from cylinder hydrogen (during irrigation, the outlet hydrogen concentration of the hydrogen-rich water is about 500 ppb, and the half-life thereof is about 3 hours), no chemical fertilizer or pesticide applied.
Field no. 7: irrigation with nanobubble hydrogen water prepared from cylinder hydrogen (during irrigation, the outlet hydrogen concentration of the hydrogen-rich water is about 1000 ppb, and the half-life thereof is about 6 hours), no chemical fertilizer or pesticide applied.
Field no. 9: irrigation with nanobubble hydrogen water prepared from cylinder hydrogen (during irrigation, the outlet hydrogen concentration of the hydrogen-rich water is about 1500 ppb, and the half-life thereof is about 8 hours), no chemical fertilizer or pesticide applied.

In field no. 3, field no. 5, field no. 7 and field no. 9, the amount of water used for hydrogen water irrigation in the growth period of strawberries accounted for 30% of the total amount of irrigation water. The method of irrigation was drip irrigation, the irrigation flow rate was 10 t/h, and the duration of each irrigation was 2 hours or more.

This embodiment is not intended to limit the number of times irrigation is performed or the way in which it is performed in the growth period of strawberries. Those skilled in the art may choose to first irrigate with hydrogen water accounting for 30% of the total amount of irrigation water on each occasion that irrigation is performed; or may choose to irrigate with hydrogen water several times in a concentrated fashion, such that the volume of hydrogen water reaches about 30% of the total amount of irrigation water for the entire growth period.

Ripe strawberries were picked; on each occasion that treatment was performed, 20 samples were taken at random, then ground into a uniform slurry, and gas mass spectrometry was used to identify volatile aromatic substances and determine the contents thereof. Research has found that a total of 54 main volatile aromatic compounds have been identified in the fruits of "Hongyan" strawberries, including alcohol substances, aldehyde substances, acid substances, ketone substances and ester substances, etc. Typical aldehyde substances include hexanal and trans-2-hexenal, ester substances include ethyl hexanoate, acid substances include hexanoic acid, alcohol substances include terpene alcohols, for example linalool and nerolidol, and ketone substances include 2,5-dimethyl-4-methoxy-3(2H)-furanone (DMMF), etc.

The contents of the main aromatic compounds in the fruits of "Hongyan" strawberries were separately determined in this embodiment. In Table 1, the contents of the abovementioned 54 main volatile alcohol substances, ester substances and ketone substances, etc. were measured separately, and added together to obtain the total content of aromatic substances. The internal standard method was used to measure the types of volatile substances mentioned above; a universal internal standard was added to the measured strawberry samples, the amount (in µg) of internal standard contained in each gram of volatile substance was computed according to peak area, and the contents of the volatile substances under test were thereby measured.

The effect of the treatment method for each group on the total content of aromatic substances in the strawberry fruits can be seen from Table 1. Compared with the use of ordinary water for irrigation treatment in field no. 1, as the hydrogen concentration in water increases, the total content of aromatic substances in strawberries also increases. The effect of nanobubble hydrogen water is more obvious, perhaps because the hydrogen in nanobubble hydrogen water has been dissolved to the greatest extent possible, and has a longer residence time in the water, so is more able to meet the demands of a long period of irrigation.

**Table 1: Total content of aromatic substances in strawberries**

| Field no. | Dissolved hydrogen concentration (ppb) | Total content of aromatic substances (µg/g) | Increase/decrease (%) relative to field no. 1 |
|---|---|---|---|
| 1 | 0 | 8.49±1.30 | -------- |
| 3 | 300 | 9.97±0.96 | 17.43 |
| 5 | 500 | 11.70±1.53 | 37.81 |
| 7 | 1000 | 13.08±0.99 | 54.06 |
| 9 | 1500 | 14.59±1.64 | 71.85 |

Aldehydes are an important type of aromatic substance in strawberries. Strawberry lipoxygenase gene *(FaLOX)* is associated with the synthesis of volatile aldehydes. The aldehyde substances measured in Table 2 include hexanal and trans-2-hexenal. It can be seen from Table 2 below that irrigation with hydrogen water can significantly increase the expression level of *FaLOX,* which exhibits the same trend of variation as the content of aldehyde substances in strawberries.

**Table 2: Content of aldehyde substances and relative expression level of lipoxygenase (FaLOX) gene in strawberries**

| Field no. | Dissolved hydrogen concentration (ppb) | Aldehyde substances (µg/g) / change (%) relative to field no. 1 | *FaLOX* / *change* (%) relative to field no. 1 |
|---|---|---|---|
| 1 | 0 | 4.20±0.96/ ------ | 1.31±0.12/ ------ |
| 3 | 300 | 6.02±1.44/ 43.33 | 4.31±0.01/ 229.01 |
| 5 | 500 | 6.07±1.32/ 44.52 | 4.42±0.01/ 237.40 |
| 7 | 1000 | 6.67±0.57/ 58.81 | 4.54±0.05/ 246.56 |
| 9 | 1500 | 7.07±2.08/ 68.33 | 4.69±1.10/ 258.02 |

Besides the above, the inventors have observed that, with regard to the contents of specific ester, alcohol and ketone compounds in strawberries and their corresponding gene expression levels, it is by no means true that hydrogen water of any concentration can have a boosting effect on the contents of these substances, despite the fact that hydrogen-rich water of low concentration can increase the total contents of aromatic substances and aldehyde substances in strawberries. Irrigation with hydrogen water of different concentrations has different effects on each type of substance; this results in differences in the contents of aromatic substances in strawberries, and also affects the completeness of strawberry flavour.

When hydrogen-rich water of low concentration is used for irrigation, the contents of some of the characteristic aromatic substances will conversely decrease relative to irrigation with ordinary water. Their contents will only exhibit a rising trend relative to the ordinary water irrigation group if hydrogen water of a higher concentration is used for irrigation for a period of time.

The contents of ester and alcohol substances in strawberries are shown in Table 3. Here, the alcohol substances include linalool and nerolidol, and the ester substances include ethyl hexanoate.

**Table 3: Contents of ester and alcohol substances in strawberries**

| Field no. | Dissolved hydrogen concentration (ppb) | Alcohol substances (µg/g) / change (%) relative to field no. 1 | Ester substances (µg/g) / change (%) relative to field no. 1 |
|---|---|---|---|
| 1 | 0 | 0.97±0.10/------ | 1.31±0.22/------ |
| 3 | 300 | 0.45±0.06/ -53.61 | 1.06±0.14/ -19.08 |
| 5 | 500 | 1.12±0.13/ 15.46 | 1.40±0.21/ 6.87 |
| 7 | 1000 | 1.34±0.07/ 38.14 | 1.54±0.19/ 17.56 |
| 9 | 1500 | 1.60±0.07/ 64.95 | 1.72±0.10/ 31.30 |

| | | | |
|---|---|---|---|
| "-" denotes a decrease | | | |

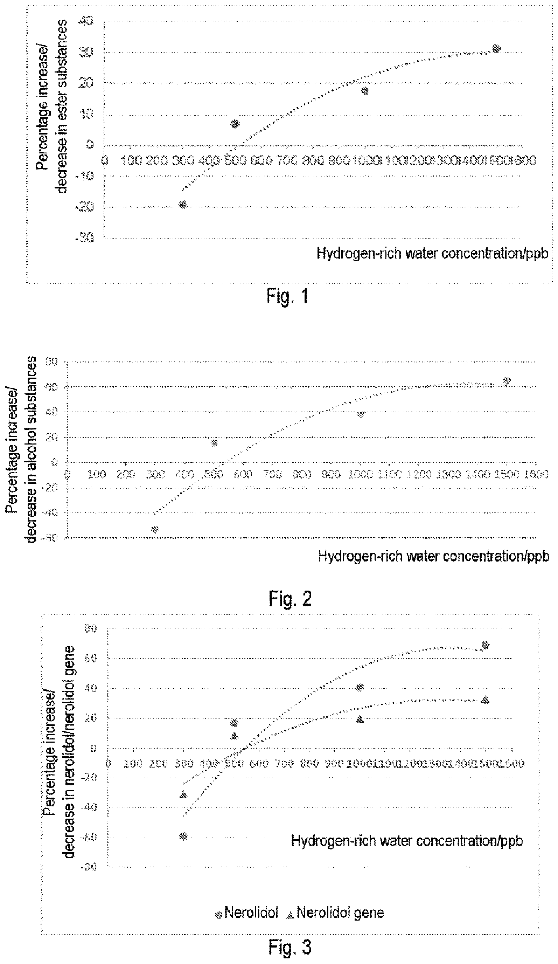

Figs. 1 and 2 show the relationship between hydrogen-rich water concentration and the percentage changes in ester substances and alcohol substances in field no. 3, field no. 5, field no. 7 and field no. 9 relative to field no. 1 in Table 3. As shown in Figs. 1 and 2, it can be seen that irrigation with hydrogen water can only effectively promote an increase in the contents of ester and alcohol substances when the outlet hydrogen water concentration is greater than 510 - 530 ppb.

In the fruits of "Hongyan" strawberries, the main characteristic aromatic substances among alcohol substances are linalool and nerolidol. Table 4 shows the variation in contents of linalool and nerolidol in the five field groups. Compared with field no. 1 irrigated with ordinary water, irrigation with hydrogen water of low concentration conversely resulted in a drop in the contents of linalool and nerolidol, and irrigation with hydrogen water of higher concentration was needed to gradually increase the contents of linalool and nerolidol. The inventors believe this might be because irrigation with hydrogen water of lower concentration causes a reduction in the amount of elemental oxygen absorbed by the root system of plants, in which case the biological activity of hydrogen is not sufficient to offset the negative effects of oxygen deficiency; therefore, compared to field no. 1, field no. 3 exhibited a phenomenon whereby the contents of linalool and nerolidol conversely fell. As the hydrogen water concentration increased, the biological activity of hydrogen become stronger, and only then did its effect in terms of boosting aromatic substances became evident.

**Table 4: Contents of linalool and nerolidol and relative expression level of nerolidol synthase gene (FaNES1) in strawberries**

| Field no. | Dissolved hydrogen concentration (ppb) | Linalool (µg/g) / change (%) relative to field no. 1 | Nerolidol (µg/g) / change (%) relative to field no. 1 | *FaNES1*/ change (%) relative to field no. 1 |
|---|---|---|---|---|
| 1 | 0 | 0.54±0.05/----- | 0.42±0.14/----- | 1.22±0.07/----- |
| 3 | 300 | 0.28±0.08/ -48.15 | 0.17±0.01/ -59.52 | 0.84±0.06/ -31.15 |
| 5 | 500 | 0.60±0.05/ 11.11 | 0.49±0.02/ 16.67 | 1.32±0.10/ 8.20 |
| 7 | 1000 | 0.70±0.04/ 29.63 | 0.59±0.03/ 40.48 | 1.46±0.05/ 19.67 |
| 9 | 1500 | 0.84±0.09/ 55.56 | 0.71±0.03/ 69.05 | 1.62±0.29/ 32.79 |

| | | | | |
|---|---|---|---|---|
| "-" denotes a decrease | | | | |

Strawberry nerolidol synthase gene (*FaNES1*) is associated with the synthesis of terpene compounds. Expression levels of *FaNES1* are shown in Table 4; the relative expression level thereof exhibits the same trend of variation as the content of nerolidol.

Fig. 3 shows the relationship between hydrogen-rich water concentration and the percentage changes in linalool, nerolidol and *FaNES1* in field no. 2, field no. 5, field no. 7 and field no. 9 relative to field no. 1 in Table 4. It can be seen from Fig. 3 that irrigation with hydrogen water can only effectively promote an increase in nerolidol and the relative expression level of nerolidol synthase gene (*FaNES1*) when the hydrogen water concentration is greater than about 540 ppb.

In addition, 2,5-dimethyl-4-methoxy-3(2H)-furanone (DMMF) is also a characteristic aromatic component of strawberries. As shown in Table 5, compared with ordinary water irrigation in field no. 1, irrigation with hydrogen water of low concentration conversely causes the content of DMMF to fall, but irrigation with hydrogen water of higher concentration can increase the content of DMMF in strawberry fruits to a certain extent. Moreover, as the concentration of nanobubble hydrogen water rises, the DMMF content rises more significantly. Strawberry O-methyltransferase gene (*FaOMT*) is a key enzyme in DMMF synthesis. The relative expression level of *FaOMT* exhibits the same trend of variation as the DMMF content. Irrigation with nanobubble hydrogen water of higher concentration is needed to increase the expression level of *FaOMT* in fruits; irrigation with hydrogen water of low concentration conversely results in a decrease in the relative expression level of *FaOMT.*

**Table 5: DMMF content and relative expression level of O-methyltransferase gene (FaOMT) in strawberries**

| Field no. | Dissolved hydrogen concentration (ppb) | DMMF(µg/g) / change (%) relative to field no. 1 | *FaOMT* / change (%) relative to field no. 1 |
|---|---|---|---|
| 1 | 0 | 0.51±0.32/----- | 3.05±0.17/----- |
| 3 | 300 | 0.18±0.01/-64.71 | 1.05±0.02/-65.57 |
| 5 | 500 | 0.52±0.17/1.96 | 3.83±0.09/25.57 |
| 7 | 1000 | 0.54±0.11/5.88 | 4.87±0.20/59.67 |
| 9 | 1500 | 0.58±0.41/13.73 | 5.91±0.37/93.77 |

| | | | |
|---|---|---|---|
| "-" denotes a decrease | | | |

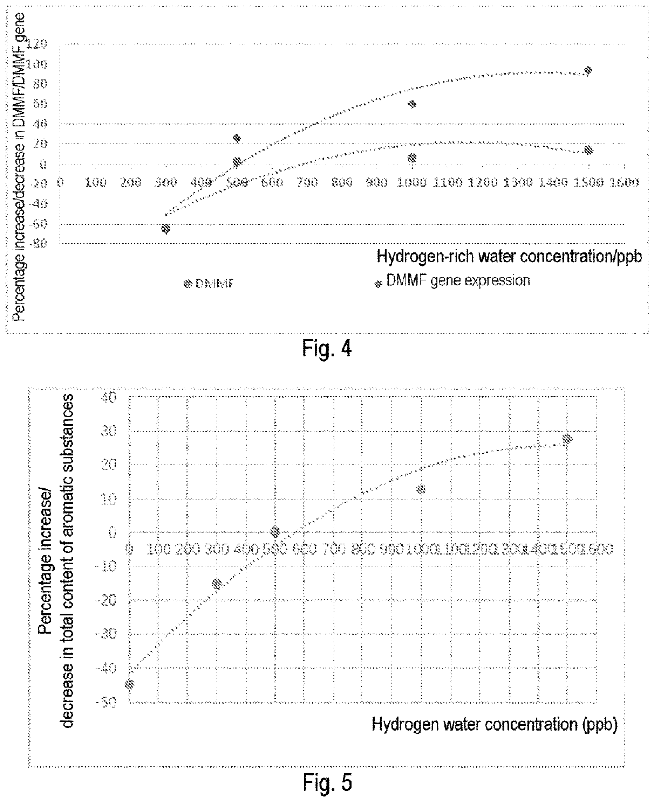

Fig. 4 shows the relationship between hydrogen-rich water concentration and the percentage changes in DMMF and the relative expression level of *FaOMT* in field no. 2, field no. 5, field no. 7 and field no. 9 relative to field no. 1 in Table 5. It can be seen from Fig. 4 that irrigation with hydrogen water can only effectively promote an increase in the relative expression level of *FaOMT* when the hydrogen water concentration is greater than 500 ppb; and irrigation with hydrogen water can only effectively promote an increase in DMMF when the hydrogen water concentration is greater than 680 ppb.

In previous research, people have often paid more attention to the growth regulator action of hydrogen or hydrogen water on crops, generally believing that it is only necessary to irrigate crops with hydrogen water in order to positively boost the growth, development and morphological formation of plants, to regulate the growth, development and nutritional quality of fruits and vegetables. However, in the course of investigating aromatic substances in strawberries, the inventors of the present invention have observed that only irrigation with hydrogen water higher than a specific concentration will manifestly promote an increase in various aromatic substances in strawberries, and only such irrigation will be able to make the flavour of strawberry fruits more complete. Making use of nanobubble technology, nanobubble hydrogen water will have a longer half-life, and can bring the biological effects of hydrogen into play more effectively.

### Embodiment 2:

This embodiment takes field production of strawberries as an example; the "Hongyan" strawberry seeds chosen were purchased at Shanghai City Seed Market. The "Hongyan" strawberry seeds were sown in fields, the area of each field being 467 square metres (about 0.7 mu). Each field was treated as follows:
Field no. 1: irrigation with ordinary water, no chemical fertilizer or pesticide applied.
Field no. 2: irrigation with ordinary water, application of the chemical fertilizers and pesticides listed below.
Field no. 4: irrigation with hydrogen-rich water prepared by electrolysis (during irrigation, the outlet hydrogen concentration of the hydrogen-rich water is about 300 ppb, and the half-life thereof is about 1 hour), application of the chemical fertilizers and pesticides listed below.
Field no. 6: irrigation with nanobubble hydrogen water prepared from cylinder hydrogen (during irrigation, the outlet hydrogen concentration of the hydrogen-rich water is about 500 ppb, and the half-life thereof is about 3 hours), application of chemical fertilizers and pesticides in the amounts listed below.
Field no. 8: irrigation with nanobubble hydrogen water prepared from cylinder hydrogen (during irrigation, the outlet hydrogen concentration of the hydrogen-rich water is about 1000 ppb, and the half-life thereof is about 6 hours), application of the chemical fertilizers and pesticides listed below.
Field no. 10: irrigation with nanobubble hydrogen water prepared from cylinder hydrogen (during irrigation, the outlet hydrogen concentration of the hydrogen-rich water is about 1500 ppb, and the half-life thereof is about 8 hours), application of the chemical fertilizers and pesticides listed below.

In field no. 2, field no. 4, field no. 6, field no. 8 and field no. 10, the amount of water used for hydrogen water irrigation in the growth period of strawberries accounted for 30% of the total amount of irrigation water. The method of irrigation was drip irrigation, the irrigation flow rate was 10 t/h, and the duration of each irrigation was 2 hours or more. This embodiment is not intended to limit the number of times irrigation is performed or the way in which it is performed in the growth period of strawberries. Those skilled in the art may choose to first irrigate with hydrogen water accounting for 30% of the total amount of irrigation water on each occasion that irrigation is performed; or may choose to irrigate with hydrogen water several times in a concentrated fashion, such that the volume of hydrogen water reaches about 30% of the total amount of irrigation water for the entire growth period.

Amounts of pesticides applied (based on the area of each field group):
First application: pendimethalin (Stomp) (herbicide) 165 ml; butachlor (herbicide) 200 ml;
Second application (10 days after first application): isoprothiolane 25 ml; yttrium 41.7 ml; prochloraz + chitosan (Micron chitin) 41.7 ml; kasugmycin (kasumin) 50 ml; pyraclostrobin 25 ml.
Third application (10 days after second application): fluazinam 83 ml; gibberellic acid 33 ml; mefenoxam + fludioxonil + azoxystrobin (spermimepyrizoil) 50 ml; zhongshengmycin 67 ml.
Fourth application (10 days after third application): tetrachlorantraniliprole (tetrachloramide) 67 ml; mefenoxam + fludioxonil + azoxystrobin (spermimepyrizoil) 50 ml; gibberellic acid 33 ml; fluazinam 83 ml.
Fifth application (7 days after fourth application): pyraclostrobin + metiram (azolidazole ether derivatives) 47 g; spirotetramat 33 ml; hymexazol 167 ml; Flowers phosphorus dynamics 167 ml; prochloraz 90 ml.
Sixth application (18 days after fifth application): prochloraz + chitosan (Micron chitin) 62 ml; bifenthrin 150 ml; acetamiprid 90 ml; chlorantraniliprole 50 ml; propamocarb hydrochloride (dimethomyl hydrochloride) 50 ml.
Seventh application (87 days after sixth application): ethirimol 50 ml; azoether fluramide 25 ml; pyrimethanil 50 ml; manntiol-Ca 66.7 ml.

Amounts of chemical fertilizers applied (based on the area of each field group):
First application: organic fertilizer 750 kg, compound fertilizer 15 kg, bacterial manure 3 kg.
Second application (52 days after first application): compound fertilizer 20 kg.

Ripe strawberries were picked; on each occasion that treatment was performed, 20 samples were taken at random, then ground into a uniform slurry, and gas mass spectrometry was used to identify volatile aromatic substances and determine the contents thereof. Research has found that a total of 54 main volatile aromatic compounds have been identified in the fruits of "Hongyan" strawberries, including alcohol substances, aldehyde substances, acid substances, ketone substances and ester substances, etc. Typical aldehyde substances include hexanal and trans-2-hexenal, ester substances include ethyl hexanoate, acid substances include hexanoic acid, alcohol substances include terpene alcohols, for example linalool and nerolidol, and ketone substances include 2,5-dimethyl-4-methoxy-3(2H)-furanone (DMMF), etc.

The contents of the main aromatic compounds in the fruits of "Hongyan" strawberries were separately determined in this embodiment. In Table 6, the contents of the abovementioned 54 main volatile alcohol substances, ester substances and ketone substances, etc. were measured separately, and added together to obtain the total content of aromatic substances. The internal standard method was used to measure the types of volatile substances mentioned above; a universal internal standard was added to the measured strawberry samples, the amount (in µg) of internal standard contained in each gram of volatile substance was computed according to peak area, and the contents of the volatile substances under test were thereby measured.

The effect of the treatment method for each group on the total content of aromatic substances in the strawberry fruits can be seen from Table 6. Compared with the use of ordinary water for irrigation treatment in field no. 1, as the hydrogen concentration in water increases, the total content of aromatic substances in strawberries also increases. The effect of nanobubble hydrogen water is more obvious, perhaps because the hydrogen in nanobubble hydrogen water has been dissolved to the greatest extent possible, and has a longer residence time in the water, so is more able to meet the demands of a long period of irrigation.

It can be seen from field no. 1 and field no. 2 that the use of chemical fertilizers and pesticides alone will result in a drop in the total concentration of aromatic substances in strawberries. Hydrogen-rich water can alleviate to a certain extent the damage caused to aromatic substances in strawberries by chemical fertilizers and pesticides.

Fig. 5 shows the relationship between hydrogen-rich water concentration and the percentage changes in field no. 2, field no. 4, field no. 6, field no. 8 and field no. 10 relative to field no. 1 in Table 6. As shown in Fig. 5, the total content of aromatic substances in strawberry fruits only begins to exceed the total content of aromatic substances in field no. 1 when the outlet hydrogen water concentration is about 580 ppb or more. This shows that if the corresponding chemical fertilizers and pesticides have been applied, the hydrogen water concentration must exceed a specific concentration in order to offset the negative effects on aromatic substances in strawberries caused by the use of chemical fertilizers and pesticides, so that the strawberries have better mouthfeel and flavour.

**Table 6: Total content of aromatic substances in strawberries**

| Field no. | Dissolved hydrogen concentration (ppb) | Total content of aromatic substances (µg/g) | Increase/decrease (%) relative to field no. 1 |
|---|---|---|---|
| 1 | 0 | 8.49±1.30 | ------ |
| 2 | 0 | 4.70±0.40 | -44.64 |
| 4 | 300 | 7.22±2.65 | -14.96 |
| 6 | 500 | 8.52±0.86 | 0.35 |
| 8 | 1000 | 9.59±1.25 | 12.96 |
| 10 | 1500 | 10.86±0.27 | 27.92 |

| | | | |
|---|---|---|---|
| "-" denotes a decrease | | | |

Aldehyde substances, ester substances, alcohol substances and ketone substances are aromatic substances with high contents in strawberry fruits. In order to study the relationships between the increase or decrease in content of each type of substance and chemical fertilizers, pesticides and hydrogen water concentration respectively, the inventors determined the content of ester substances (including ethyl hexanoate), the content of the characteristic fragrance component of strawberries 2,5-dimethyl-4-methoxy-3(2H)-furanone (DMMF), and the relative expression level of a key enzyme in DMMF synthesis, namely strawberry O-methyltransferase gene (*FaOMT*)*.* The results are shown in Table 7.

**Table 7: Content of ester substances, DMMF content and relative expression level of FaOMT in strawberries**

| Field no. | Dissolved hydrogen concentration (ppb) | Ester substances (µg/g) / change (%) relative to field no. 1 | DMMF (µg/g) / change (%) relative to field no. 1 | *FaOMTI* change (%) relative to field no. 1 |
|---|---|---|---|---|
| 1 | 0 | 1.31±0.22/----- | 0.51±0.32/----- | 3.05±0.17/----- |
| 2 | 0 | 1.10±0.13/-16.03 | 0.20±0.01/-60.78 | 1.00±0.05/-67.21 |
| 4 | 300 | 1.00±0.28/-23.66 | 0.26±0.16/-49.02 | 1.01±0.10/-66.89 |
| 6 | 500 | 1.11±0.16/-15.27 | 0.31±0.09/-39.22 | 1.58±0.11/-48.20 |
| 8 | 1000 | 1.13±0.12/-13.74 | 0.33±0.20/-35.29 | 1.88±0.13/-38.36 |
| 10 | 1500 | 1.17±0.20/-10.69 | 0.34±0.01/-33.33 | 2.24±0.22/-26.56 |

Strawberry O-methyltransferase gene (*FaOMT*) is a key enzyme in DMMF synthesis. The expression of *FaOMT* exhibits the same trend of variation as the DMMF content. In the case where chemical fertilizers and pesticides are applied, hydrogen water can alleviate to a certain extent the suppressing action of the chemical fertilizers and pesticides on volatile ester substances and DMMF in strawberry fruits. However, even with hydrogen water of as high a concentration as 1500 ppb, the contents of ester substances and DMMF are still reduced compared with field no. 1 where no pesticides or chemical fertilizers are applied. If it is desired to maintain the contents of certain ester substances and DMMF, the amounts of chemical fertilizers and pesticides used can be reduced, so that the ratio of the concentration of hydrogen in the hydrogen-rich water to the amount of chemical fertilizer applied is greater than a specific value. For example, in this embodiment, the total amount of chemical fertilizers applied is 788 kg; if the amount of chemical fertilizers applied is reduced and the concentration of hydrogen water is correspondingly increased, the hydrogen water can remedy the effect of the reduction in chemical fertilizers on the yield or single fruit weight and can also promote an increase in the contents of certain ester substances and DMMF to a greater extent.

The inventors then determined the content of aldehyde substances (including hexanal and trans-2-hexenal), the relative expression level of strawberry lipoxygenase gene *(FaLOX)* associated with the synthesis of volatile aldehydes, and the content of the characteristic aldehyde substance trans-2-hexenal in the strawberry fruits; the results are shown in Table 8.

**Table 8: Aldehyde substances, relative expression level of FaLOX and trans-2-hexenal content in strawberries**

| Field no. | Dissolved hydrogen concentration (ppb) | Aldehyde substances (µg/g) /change (%) relative to field no. 1 | *FaLOX*/ change (%) relative to field no. 1 | Trans-2-hexenal (µg/g) / change (%) relative to field no. 1 |
|---|---|---|---|---|
| 1 | 0 | 4.20±0.96/---- | 1.31±0.12/---- | 3.13±0.97/---- |
| 2 | 0 | 2.33±0.062/ -44.52 | 1.00±0.02/ -23.66 | 1.68±0.17/ -46.33 |
| 4 | 300 | 4.09±1.70/ -2.62 | 1.70±0.01/ 29.77 | 3.46±1.52/ 10.54 |
| 6 | 500 | 4.22±0.86/ 0.48 | 1.85±0.02/ 41.22 | 3.50±0.72/ 11.82 |
| 8 | 1000 | 4.38±1.55/ 4.29 | 2.15±0.03/ 64.12 | 3.65±1.03/ 16.61 |
| 10 | 1500 | 4.62±0.34/ 10.00 | 2.40±0.23/ 83.21 | 3.78±0.36/ 20.77 |

| | | | | |
|---|---|---|---|---|
| "-" denotes a decrease | | | | |

The use of chemical fertilizers and pesticides will lower the content of aldehyde substances and the expression of strawberry lipoxygenase gene in strawberry fruits. Hydrogen with gradually increasing concentration in irrigation water offsets the negative effects of chemical fertilizers and pesticides to a certain extent. The relative expression level of strawberry lipoxygenase gene exhibits a rising trend overall, there will be a significant increase in flavour substances in strawberries, and this exhibits the same trend of variation as the content of aldehyde substances.

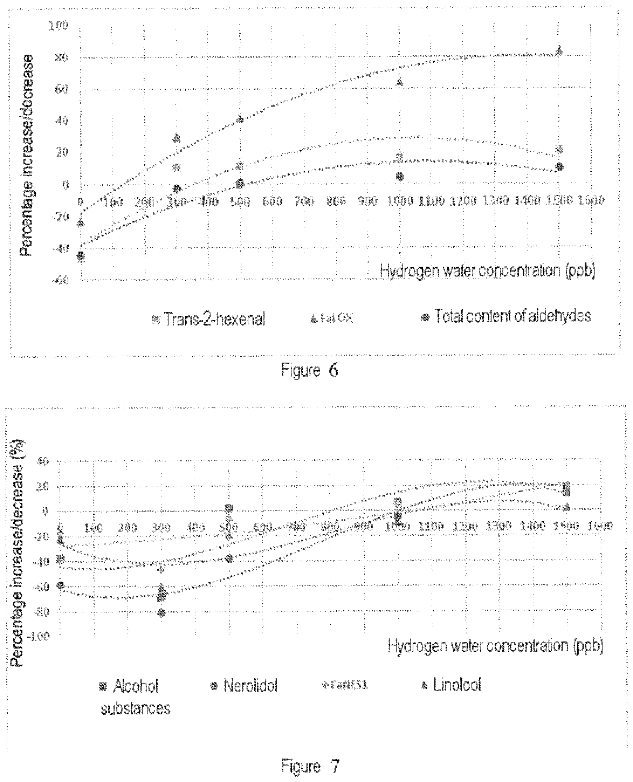

Fig. 6 shows the relationship between hydrogen-rich water concentration and the percentage changes in aldehyde substances, *FaLOX* and trans-2-hexenal relative to field no. 1 in Table 8. As shown in Fig. 6, the contents of aldehyde substances, *FaLOX* and trans-2-hexenal exhibit a rising trend overall, and the negative effects of chemical fertilizers and pesticides on the contents of these types of substance begin to be completely offset at hydrogen water concentrations of about 500 ppb, 120 ppb and 350 ppb respectively.

The inventors of the present invention have observed that, with regard to aromatic substances with high contents in strawberries, it is by no means true that irrigation with hydrogen water of any concentration can effectively offset the suppressing effect of pesticides and chemical fertilizers on the contents thereof. Irrigation with hydrogen water of different concentrations has different effects on each type of substance, resulting in completeness of contents of aromatic substances and different flavours in strawberries.

In the fruits of "Hongyan" strawberries, the main characteristic aromatic substances among alcohol substances are linalool and nerolidol. Strawberry nerolidol synthase gene (*FaNES1*) is associated with the synthesis of terpene compounds. As shown in Table 9, the use of chemical fertilizers and pesticides will lower the contents of alcohol substances, linalool and nerolidol in strawberry fruits, and will also lower the relative expression level of nerolidol synthase gene (*FaNES1*)*,* and this relative expression level exhibits the same trend of variation as the content of nerolidol. Only hydrogen water of a specific concentration can completely offset the suppressing effect of chemical fertilizers and pesticides on these types of aromatic substance in strawberry fruits.

**Table 9: Contents of alcohol substances, linalool and nerolidol in strawberries**

| Field no. | Dissolved hydrogen concentrat ion (ppb) | Linalool (µg/g) / change (%) relative to field no. 1 | Nerolidol (µg/g) / change (%) relative to field no. 1 | *FaNES1* change (%) relative to field no. 1 | Alcohol substances (µg/g) /change (%) relative to field no. 1 |
|---|---|---|---|---|---|
| 1 | 0 | 0.54±0.05/----- | 0.42±0.14/----- | 1.22±0.07/ ----- | 0.97±0.10/ ----- |
| 2 | 0 | 0.42±0.07/ -22.22 | 0.17±0.01/ -59.52 | 1.00±0.03/-18.03 | 0.60±0.23/-38.14 |
| 4 | 300 | 0.21±0.06/ -61.11 | 0.08±0.04/ -80.95 | 0.65±0.02/ -46.72 | 0.30±0.09/ -69.07 |
| 6 | 500 | 0.44±0.02/ -18.52 | 0.26±0.01/ -38.10 | 1.14±0.03/ -6.56 | 0.99±0.08/ 2.06 |
| 8 | 1000 | 0.49±0.03/ -9.26 | 0.40±0.02/ -4.76 | 1.27±0.06/ 4.10 | 1.03±0.11/ 6.19 |
| 10 | 1500 | 0.55±0.06/ 1.85 | 0.50±0.07/ 19.05 | 1.45±0.27/ 18.85 | 1.1±0.09/ 13.40 |

| | | | | | |
|---|---|---|---|---|---|
| "-" denotes a decrease | | | | | |

Fig. 7 shows the relationship between hydrogen-rich water concentration and the percentage changes in field no. 2, field no. 4, field no. 6, field no. 8 and field no. 10 relative to field no. 1 in Table 9. As shown in Fig. 7, the contents of alcohol substances, *FaNES1,* linalool and nerolidol exhibit a rising trend overall, and the negative effects of chemical fertilizers and pesticides on the contents of these types of substance begin to be completely offset at hydrogen water concentrations of about 800 ppb, 1000 ppb, 1000 ppb and 1000 ppb respectively. At a low hydrogen concentration, such as 300 ppb, the contents of alcohol substances, *FaNES1,* linalool and nerolidol are at their lowest values, meaning that hydrogen at a low concentration might have a certain suppressing effect on the synthesis of specific compounds; this suppressing effect is added to the suppressing effect of chemical fertilizers and pesticides, resulting in a considerable reduction in the synthesis of these compounds.

It can be seen from the above results that after exceeding a certain concentration, the hydrogen water provided in the present invention has an obvious boosting effect on the manifestation of aromatic substances in strawberries, and thereby offsets the negative effects of the use of chemical fertilizers and pesticides on aromatic substances in strawberries. Hydrogen water of different specific concentrations affects various aromatic substances in strawberries to different extent, so hydrogen water of a suitable concentration needs to be chosen in order to continue to maintain or increase the flavour of strawberries while completely offsetting the damage done to flavour by chemical fertilizers and pesticides.

Soil needs to absorb enough nourishment in order to replenish nutrients, while pesticides can eliminate various diseases and insect pests that affect the yield or appearance of crops. Therefore, it would be very difficult to completely eliminate the use of pesticides and chemical fertilizers if crops yields are to be pursued and diseases and insect pests are to be reduced. The above patterns and conclusions observed by the inventors of the present invention can provide some pointers for the healthy development of field agriculture. The method of irrigating fields with hydrogen-rich water is especially suitable for increasing the expression of aromatic substances in fruits and vegetables, in particular in berries, while the amounts of pesticides and chemical fertilizers applied are suitably reduced. The biological effects of hydrogen at high concentration can be brought into play while meeting the normal demand of fruits and vegetables for water, to achieve the objectives of ensuring the yield of fruits and vegetables, increasing the content of aromatic substances in fruits and vegetables, and increasing the fragrance of fruits and vegetables.

The embodiments described herein are merely preferred specific embodiments of the present invention, which are only intended to explain the technical solution of the present invention without limiting the present invention. All technical solutions that can be obtained by those skilled in the art by logical analysis, reasoning or limited experiment according to the concept of the present invention should fall within the scope of the present invention.

## Claims

1. A method for using hydrogen-rich water to increase the content of aromatic substances in fruits and vegetables, **characterized in that** an outlet hydrogen concentration of hydrogen-rich water is kept no lower than a set value within a period of time for which a field is irrigated with hydrogen-rich water.

2. The method as claimed in claim 1, **characterized in that** the period of time for which the field is irrigated is at least 2 hours.

3. The method as claimed in claim 1, **characterized in that** the amount of the hydrogen-rich water used to irrigate the field accounts for 30% or more of the total amount of water used to irrigate the field.

4. The method as claimed in claim 1, **characterized in that** the hydrogen-rich water is nanobubble hydrogen water.

5. The method as claimed in claim 1, **characterized in that** the set value of the outlet hydrogen concentration of the hydrogen-rich water is 500 ppb, preferably 600 ppb, more preferably 700 ppb, and most preferably 1000 ppb.

6. The method as claimed in claim 1, **characterized in that** the aromatic substances comprise ester substances, ketone substances and alcohol substances.

7. A method for using hydrogen-rich water to increase gene expression of aromatic substances in fruits and vegetables, **characterized in that** an outlet hydrogen concentration of hydrogen-rich water is kept no lower than a set value within a period of time for which a field is irrigated with hydrogen-rich water.

8. A method for using hydrogen-rich water, pesticide and chemical fertilizer in combination to maintain or increase the content of aromatic substances in fruits and vegetables, **characterized in that** an outlet hydrogen concentration of hydrogen-rich water is kept no lower than a set value within a period of time for which a field is irrigated with hydrogen-rich water;
wherein chemical fertilizer and pesticide are applied to the field in the following doses:
one of, or a combination of more than one of, the following pesticides is sprayed within a growth period of the fruits and vegetables:
pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40 - 50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu;
the chemical fertilizer application plan is as follows: organic fertilizer 1000 - 1100 kg/mu, compound fertilizer 70 - 80 kg/mu, bacterial manure 4 - 5 kg/mu.

9. The method as claimed in claim 8, **characterized in that** the set value is 500 ppb, preferably 700 ppb, more preferably 1000 ppb, and most preferably 1500 ppb.

10. The method as claimed in claim 8, **characterized in that** the period of time for which the field is irrigated is at least 2 hours.

11. The method as claimed in claim 8, **characterized in that** the hydrogen-rich water is nanobubble hydrogen water.

12. The method as claimed in claim 1, **characterized in that** the aromatic substances comprise ester substances, ketone substances and alcohol substances.

13. A method for using hydrogen-rich water, pesticide and chemical fertilizer in combination to maintain or increase the content of aromatic substances in fruits and vegetables, **characterized in that**, within a period of time for which a field is irrigated with hydrogen-rich water, the ratio of an outlet hydrogen concentration of hydrogen-rich water to an amount of chemical fertilizer applied is kept greater than 0.63 ppb/kg, preferably greater than 0.89 ppb/kg, more preferably greater than 1.27 ppb/kg, and most preferably greater than 1.90 ppb/kg.

14. The method as claimed in claim 13, **characterized in that** one of, or a combination of more than one of, the following pesticides is sprayed within a growth period of the fruits and vegetables:
pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40 - 50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu.

15. A method for using hydrogen-rich water, pesticide and chemical fertilizer in combination to maintain or increase gene expression of aromatic substances in fruits and vegetables, **characterized in that** an outlet hydrogen concentration of hydrogen-rich water is kept no lower than a set value within a period of time for which a field is irrigated with hydrogen-rich water;
wherein chemical fertilizer and pesticide are applied to the field in the following doses:
one of, or a combination of more than one of, the following pesticides is sprayed within a growth period of the fruits and vegetables:
pendimethalin (Stomp) at 200 - 250 ml/mu, butachlor at 280 - 300 ml/mu, isoprothiolane at 30 - 40 ml/mu, yttrium at 50 - 60 ml/mu, prochloraz + chitosan (Micron chitin) at 55 - 60 ml/mu, kasugmycin (kasumin) at 70 - 80 ml/mu, pyraclostrobin at 30 - 40 ml/mu, fluazinam at 230 - 250 ml/mu, gibberellic acid at 40-50 ml/mu, mefenoxam + fludioxonil + azoxystrobin (spermimepyrizoil) at 70 - 80 ml/mu, zhongshengmycin at 90 - 100 ml/mu, tetrachlorantraniliprole (tetrachloramide) at 90 - 100 ml/mu, mefenoxam + fludioxonil + azoxystrobin (spermimepyrizoil) at 70 - 80 ml/mu, gibberellic acid at 40 - 50 ml/mu, pyraclostrobin + metiram (azolidazole ether derivatives) at 60 - 70 g/mu, spirotetramat at 40 - 50 ml/mu, hymexazol at 220 - 250 ml/mu, Flowers phosphorus dynamics at 220 - 250 ml/mu, prochloraz at 120 - 130 ml/mu, prochloraz + chitosan (Micron chitin) at 80 - 90 ml/mu, bifenthrin at 210 - 230 ml/mu, acetamiprid at 120 - 130 ml/mu, chlorantraniliprole at 70 - 80 ml/mu and/or propamocarb hydrochloride (dimethomyl hydrochloride) at 70 - 80 ml/mu;
the chemical fertilizer application plan is as follows: organic fertilizer 1000 - 1100 kg/mu, compound fertilizer 70 - 80 kg/mu, bacterial manure 4 - 5 kg/mu.
